# NEUE EUROPÄISCHE PATENTSCHRIFT

(11) **EP 1 107 775 B2**
(45) Veröffentlichungstag und Bekanntmachung des Hinweises auf die Entscheidung über den Einspruch: **14.01.2009**
(45) Hinweis auf die Patenterteilung: 07.05.2003
(21) Anmeldenummer: 99932748.9
(22) Anmeldetag: 29.06.1999
(51) Int. Cl.: A61P 17/00, A61P 17/06, A61P 15/00, A61P 37/08

(54) **PYRROLIZIDINALKALOIDFREIE PETASITES ENTHALTENDE ZUSAMMENSETZUNG**
COMPOSITION CONTAINING PYRROLIZIDINE-ALKALOID-FREE PETASITES
COMPOSITION CONTENANT DES PETASITES EXEMPTS DE PYRROLIZIDINALCALOIDES

(30) Priorität: 26.08.1998 DE 19838848
(43) Veröffentlichungstag der Anmeldung: 20.06.2001
(73) Patentinhaber: Max-Zeller Söhne AG, 8590 Romanshorn (CH)
(72) Erfinder: KOCH, Volkmar, D-86919 Utting/Ammersee (DE); RITTINGHAUSEN, Reiner, D-82279 Eching/Ammersee (DE)
(74) Vertreter: Ritscher, Thomas
(86) Internationale Anmeldenummer: PCT/EP1999/004484
(87) Internationale Veröffentlichungsnummer: WO 2000/012107

(56) Entgegenhaltungen:
- EP-B1- 0 391 504
- EP-B1- 0 508 330
- DE-A- 4 141 749
- DE-A1- 19 702 168
- DE-C- 3 910 831
- DATABASE WPI Section Ch, Week 198830 Derwent Publications Ltd., London, GB; Class B05, AN 1988-211090 XP002118192 & RO 93 835 A (DIRECT SANITARA JUDET), 29. Februar 1988 (1988-02-29)

## Beschreibung

Die vorliegende Erfindung betrifft die Verwendung einer pharmazeutischen Zusammensetzung basierend auf Petasitesextrakt, ohne hepatotoxischer, carcinogener, zytostatischer und/oder mutagener Wirkung, sowie Verfahren zu ihrer Herstellung.

Petasitesextrakte werden aus den Wurzeln der Pestwurz isoliert und haben eine krampflösende und analgetische Wirkung. Diese Wirkung der Pestwurz war bereits Hippokrates, Galen und Paracelsus bekannt. Bei den derzeit verwendeten pharmazeutischen Präparaten aus Petasitesextrakt handelt es sich insbesondere um zähflüssige Extrakte von Rhizoma Petasites, d.h. Extrakte aus dem Wurzelstock der Pestwurz, die einen unangenehmen,
bitteren Geschmack aufweisen, nicht gut resorbierbar sind, und so nach Verabreichung eine leicht verzögerte pharmazeutische Wirkung aufweisen.

Außerdem enthält die Pestwurz neben weiteren Sesquiterpenen auch Eremophilane und Pyrrolizidinalkaloide. Den Pyrrolizidinalkaloiden ist das Pyrrolizidin-Grundgerüst gemeinsam. Sie sind weltweit in höheren Pflanzen verbreitet. So findet man Pyrrolizidinalkaloide und ihre N-Oxide unter anderem in den Gattungen der Petasites. Die in den Pflanzenteilen der Gattung Petasites enthaltenen Pyrrolizidinalkaloide zeichnen sich durch erhebliche hepatotoxische, carcinogene und mutagene, aber auch zytostatische Eigenschaften aus. Die Toxizität der Pyrrolizidinalkaloide ist an bestimmte Strukturgruppen gebunden, die folgende Strukturmerkmale aufweisen:
- Doppelbindungen in der 1,2-Stellung des Pyrrolizidin-Ringes;
- Veresterungen mindestens der primären Hydroymethylgruppe mit einer C₅ oder C₆-Carbonsäure;
- Verzweigung der Alkylseiten-Kette mit mindestens einer der Necinsäuren.
Die höchste Toxizität und Cancerogenität besitzen die cyclischen Diester. Die Pyrrolizidinalkaloide werden nach peroraler Aufnahme rasch resorbiert, ihre N-Oxide erst nach Reduktion durch die Darmflora. Bei der Metabolisierung in der Leber werden die Pyrrolizidinalkaloide durch mischfunktionelle Oxidasen in sehr toxische Pyrrol-Derivate umgewandelt. Diese sind sehr reaktiv und alkylieren unter physiologischen Bedingungen nukleophile Gruppen der DNA, wie Amino-, Thiol- und Hydroxy-Gruppen.

In der Phytotherapie wurden Pyrrolizidinalkaloid-haltige Arzneimittelpflanzen seit langem als Naturheilmittel verwendet. Da das Bundesgesundheitsamt (BGA) alle Arzneipflanzen und ihre Präparate, die toxische Pyrrolizidinalkaloide enthalten, als gesundheitlich bedenklich und potentiell krebsauslösend eingestuft hat, wurde in Folge dessen intensiv daran gearbeitet, pharmazeutische Petasitesextrakte zur Verfügung zu stellen, die einen deutlich verringerten Gehalt an Pyrrolizidinalkaloiden aufweisen.

So werden in der DE 39 10 831, DE 41 11 141, DE 41 41 749 Verfahren beschrieben, mit deren Hilfe sich der Gehalt an Pyrrolizidinalkaloiden im Extrakt auf unter 5 ppm, bestenfalls sogar auf unter 0,1 ppm absenken läßt, ein wirklich Pyrrolizidinalkaloid-freier Petasitesextrakt läßt sich nach diesen Verfahren aber nicht erhalten.

Aufgabe der vorliegenden Erfindung ist es, die Verwendung von pharmazeutischen Zusammensetzungen basierend auf Petasitesextrakt ohne hepatotoxischer, carcinogener, zytotostatischer und/oder mutagener Wirkung zur Verfügung zu stellen.

Eine weitere Aufgabe der Erfindung ist es, Verfahren zur Herstellung von pharmazeutischen Zusammensetzungen basierend auf Petasitesextrakt ohne hepatotoxischer, carcinogener, zytotostatischer und/oder mutagener Wirkung zur Verfügung zu stellen.

Eine weitere Aufgabe der Erfindung ist es, die Verwendung von pharmazeutischen Zusammensetzungen zur Herstellung von Petasitespräparaten ohne hepatotoxischer, carcinogener, zytostatischer und/oder mutagener Wirkung mit einer schnellen und/oder verbesserten Wirkung zur Verfügung zu stellen.

Die Aufgabe der vorliegenden Erfindung wird durch die Verwendung einer pharmazeutischen Zusammensetzung, basierend auf Petasitesextrakt gemäß Anspruch 1 gelöst, wobei der Petasitesextrakt aus der Pflanze und/oder Pflanzenteilen der Gattung Petasites zur Herstellung eines Arzneimittels zur Behandlung, von Ekzemen und/oder von Psoriasis gewonnen wird, der Petasitesextrakt frei von Pyrrolizidinalkaloiden ist, und wobei dieser pharmazeutischen Zusammensetzung gegebenenfalls Magnesium und/oder weitere pharmazeutisch und/oder physiologisch wirksame Stoffe zugesetzt sind.

Die verfahrensbezogene Aufgabe der Erfindung wird durch ein Extraktionsverfahren gemäß den Ansprüchen 12 und 13 gelöst, wobei der Rohextrakt einer anschließenden Nachbehandlung im wäßrigen Medium mit einem pH-Wert < 7, vorzugsweise ≤ 4, unterzogen wird.

Weitere erfindungsgemäß vorteilhafte Ausführungsformen sind in den Unteransprüchen und in den Beispielen angegeben.

Im Stand der Technik wurde bisher der Petasitesextrakt aus dem Wurzelstock der Pestwurz gewonnen. Es hat sich nunmehr als vorteilhaft erwiesen, den Extrakt aus Pflanzen und/oder Pflanzenteilen von Petasites hybridus, Petasites albus, Petasites japonicus, Petasites paradoxus, und/oder Petasites spurius zu gewinnen, wobei vorzugsweise die unterirdischen Pflanzenteile zur Herstellung des Extrakts verwendet werden. Die Verwendung nicht nur der unterirdischen Pflanzenteile zur Herstellung des Extrakts, sondern auch anderer Pflanzenteile hat den Vorteil, daß man eine wesentlich höhere Ausbeute an Petasitesextrakt bezogen auf die gesamte Pflanze erhält.

Neben den bereits bekannten Destraktionsverfahren mit überkritischem CO₂, wie z.B. in der EP 0 392 504 und der EP 0 547 465 beschrieben, lassen sich erfindungsgemäße Petasitesextrakte auch mit anderen Destraktionsmitteln herstellen. Wegen der Temperaturempfindlichkeit der Naturstoffextrakte sind dabei solche Lösungsmittel auszuwählen, welche bereits bei Temperaturen von unter 100 °C, vorzugsweise bei ≤50 °C, in den überkritischen Zustand überführt werden können, und welche ein ausreichendes Lösungsvermögen für lipophile Petasiteswirkstoffe aufweisen. Geeignete Gase umfassen beispielsweise Ethylen (kritische Temperatur: 9,95 °C) und Ethan (kritische Temperatur: 32,25 °C). Geeignete Bedingungen für die Destraktion mit Ethylen und Ethan von Petasin aus Pestwurz-Pflanzenteilen liegen bei Drücken von 1-50 MPa, vorzugsweise von 8-30 MPa, und Temperaturen von 10-50 °C. Die nach diesen Verfahren gewonnenen Roh-Petasitesextrakte enthalten je nach Vortrocknungsgrad des Ausgangsmaterials bis zu 30 % Wasser, welches nachfolgend abgetrennt werden muß. Gegebenenfalls kann der Roh-extrakt mit der ein- bis zehnfachen Menge an Wasser, vorzugsweise mit einem pH-Wert ≤ 7, behandelt und nachfolgend mit üblichen Methoden getrocknet werden.

Alternativ dazu konnten gute Alkaloidabreicherungen auch durch Einpressen von Wasser in das komprimierte Lösungsmittel-Extraktgemisch im Abscheiderbereich der Extraktionsanlage erzielt werden. Dazu werden in die Abscheideeinrichtung der Extraktionsanlage vor der Dekompression des Lösemittel-Extraktgemisches ca. 10-50 Gew.-% Wasser bezogen auf die abzuscheidende Extraktmenge eingepresst und anschließend dekomprimiert. Die so erhaltenen Extrakte wiesen nach Abtrennung der Wasserphase Pyrrolizidinalkaloidgehalte unter 0,1 ppm auf.

Überraschenderweise hat sich nun auch gezeigt, daß anstelle von Destruktionen mit Gasen im überkritischen Zustand geeignete Rohextrakte von Petasites auch durch Extraktion mit unter hohem Druck verflüssigten Gasen bei unterkritischen Bedingungen erhalten werden.

Als besonders gut geeignet für die Petasitesextraktion bei unterkritischen Bedingungen hat sich druckverflüssigtes Propangas erwiesen. Propan (kritische Temperatur: 97°C) kann bei Raumtemperatur unter hohem Druck verflüssigt werden (kritischer Druck: ca. 42 MPa). In geeigneten Autoklaven-Reaktoren wird dazu nach üblichen Methoden zerkleinertes Petasites-Pflanzenmaterial einer Feststoffextraktion mit verflüssigtem Propangas unterworfen. Auch hier ist die gezielte Anreicherung der Extraktionsgutes mit bis zu 40 Gew.-% Wasser zur verbesserten Pyrrolizidinalkaloidabtrennung vorteilhaft einsetzbar, wie auch die nachträgliche Einpressung von Wasser in das komprimierte Propan-Extraktgemisch.

Das Propan-Extraktionsverfahren kann im Temperaturbereich von 15-40 °C durchgeführt werden, bevorzugt bei 20-30 °C, und besonders bevorzugt bei Raumtemperatur. Die angewandten Extraktionsdrücke liegen im Bereich von 5 bis 20 MPa, bevorzugt bei 10 bis 15 MPa und besonders bevorzugt bei etwa 14 MPa, also meist deutlich unterhalb von typischen Destraktionsdrücken. Die nach Abdampfen des Lösungsmittels erhaltenen Rohextrakte enthalten einen teilweise emulgierten Restwassergehalt von bis zu 20 % und weisen überraschenderweise einen gegenüber dem Ausgangsmaterial deutlich abgereicherten, sehr geringen Anteil an Pyrrolizidinalkaloiden auf. Überstehendes Wasser kann durch Abschöpfen oder Dekantieren abgetrennt werden. Die geringe Mengen an wasserlöslichen Pyrrolizidinalkaloiden im Rohextrakt können durch wässrige Nachbehandlung, vorzugsweise pH < 7 und anschließende Phasentrennung, wie unten beschrieben, vollständig abgetrennt werden. Weiterhin kann der Extrakt mit Hilfe üblicher Trocknungsmittel wie z.B. Natriumsulfat, Magnesiumsulfat, sowie Molsieb und dergleichen, getrocknet und von emulgiertem Wasser befreit werden.

Der Vorteil dieses erfindungsgemäßen Verfahrens liegt in der möglichen Anwendung niedrigerer Temperaturen, beispielsweise Extraktion bei Raumtemperatur, und den geringeren Drücken, die sowohl eine kostengünstige wie auch schonende Extraktion der empfindlichen Petasitesextrakte gewährleisten.

Ein weiterer Vorteil, insbesondere gegenüber den üblichen überkritischen CO₂-Destraktionsverfahren, besteht in den günstigeren Lösungseigenschaften des flüssigen Propans begründet. Da Propan als Kohlenwasserstoff die lipophilen Petasine deutlich besser zu lösen vermag als die hydrophilen Pyrrolizidinalkaloide, ist die Trennung beider Komponenten besser und die Abreicherung der unerwünschten Alkaloide aus dem Petasites-Material deutlich vollständiger. Die durch unterkritische Propanextraktion gewonnenen Rohextrakte weisen in der Regel einen Restalkaloidgehalt von deutlich unter 0,1 ppm auf. Weiterhin sind die erzielbaren Gesamtausbeuten an Rohextrakt unter vergleichbaren Bedingungen insgesamt höher.

Die erfindungsgemäße unterkritische Propanextraktion kann in üblichen Autoklaven-Reaktoren diskontinuierlich oder halbkontinuierlich durchgeführt werden, welche als Ein- oder Mehrkörper-Extraktionsanlagen konzipiert sein können. Reaktoren mit kontinuierlicher Extraktabscheidung unter Rückführung des Propans in den Extraktionsautoklaven sind dabei aus Kostengründen und unter umwelttechnischen Gesichtspunkten zu bevorzugen. Daneben kann die Extraktion auch in einfachen Druckreaktoren in einem oder mehreren sukzessiven Schritten durchgeführt werden.

Der Wasser enthaltende Extrakt läßt sich auch direkt mit einem üblichen Trocknungsmittel, vorzugsweise Na₂SO₄, trocknen, ohne daß der Extrakt mit Wasser ausgeschüttelt wird. Dazu wird überstehendes Wasser abgetrennt und dem Extrakt das Trocknungsmittel in der erforderlichen Menge zugesetzt. Das Trocknungsmittel wird dann nach im Stand der Technik üblichen Verfahren abgetrennt, beispielsweise mittels Filtration oder Zentrifugation.

Der Extrakt kann zur Entfernung von beispielsweise Pyrrolizidinalkaloiden mit der 0,01-10fachen, vorzugsweise 0,1-10fachen Menge Wasser bezogen auf den Extrakt in einen kontinuierlichen oder diskontinuierlichen Extraktionsverfahren behandelt werden.

Optional wird der Extrakt mindestens einmal mit der 0,1- bis zehnfachen Menge an Wasser mit einem pH-Wert < 7, vorzugsweise ≤ 4, besonders bevorzugt pH ≤ 1, ausgeschüttelt, die Säurephase abgetrennt, und der Extrakt durch mehrmaliges Waschen auf einen pH-Wert von 3-7 gebracht. Als Säure wird vorzugsweise eine Mineralsäure, besonders bevorzugt Schwefelsäure mit einer Normalität von 0,0001 bis 1, vorzugsweise von 0,1 bis 1 verwendet.

Mit Hilfe eines Trocknungsmittels, vorzugsweise Na₂SO₄, und/oder einer Zentrifuge kann der Wassergehalt des Petasitesextrakt auf deutlich unter 1 Gew.-% abgesenkt werden.

Alternativ oder zusätzlich können die Rohextrakte mit einem geeigneten Adsorptionsmittel, wie zum Beispiel Kieselgel, Aluminiumoxid, Aluminosilikat, Zeolith, Zirkoniumoxid, Titanoxid und Molsieb, aufgenommen und gegebenenfalls unter Zusatz von Wasser, vorzugsweise Wasserdampf, ein weiteres mal unter Destraktions- oder Extraktionsbedingungen extrahiert werden. Anschließend wird wie oben beschrieben entwässert, oder überstehendes Wasser vom Extrakt getrennt, beispielsweise durch vorsichtiges Ablassen des Extrakts, wobei die überstehende wäßrige Phase anschließend verworfen wird.
Vorteilhaft läßt sich die Phasentrennung Wasser/Extrakt mittels Ultraschall bewirken. Durch Einwirkung von Ultraschall wird die beschleunigte Ausbildung einer scharfen Phasengrenze ermöglicht.

So erhaltene Extrakte weisen einen Pyrrolizidinalkaloidgehalt von ≤ 0,1 ppm auf, wenn der Gehalt im Rohextrakt bis zu 0,5 ppm betrug. In einem weiteren Nachbehandlungsschritt wird der Extrakt wenigstens 2 mal, vorzugsweise 4 bis 6 mal, im Verhältnis von 1 Teil Extrakt und 2 Teilen bidestilliertem Wasser, bei einer Wassertemperatur von wenigstens 40°C, vermischt. Anschließend werden die Phasen getrennt und im letzten Schritt emulgiertes Restwasser durch Zugabe von üblichen Trocknungsmitteln, vorzugsweise Natriumsulfat gebunden. Das mit Wasser beladene Trocknungsmittel wird abzentrifugiert.

Zur Bestimmung des Pyrrolizidinalkaloidgehaltes im Spurenbereich kann ein ELISA herangezogen werden, welcher im Vergleich zur GC-MS-Kopplung üblicherweise um den Faktor 1000 empfindlicher ist. Es konnte in erfindungsgemäß hergestellten Petasitesextrakten in keinem Fall Pyrrolizidinalkaloide gefunden werden.

Die nach dem erfindungsgemäßen Verfahren hergestellten Petasitesextrakte enthalten etwa 40 % Petasine (berechnet als Isopetasin) und können ohne weitere Aufbereitung in pharmazeutischen Zusammensetzungen eingesetzt werden.

Eine vorteilhafte Ausführungsform der Erfindung umfaßt die Verwendung von pharmazeutischen Zusammensetzungen basierend auf Petasitesextrakt mit einem Gehalt an Magnesium, vorzugsweise
mit einem Gehalt von >0 - 500 mg, besonders bevorzugt 10 - 250 mg und noch bevorzugter 50 - 100 mg, bezogen auf die Gesamtzusammensetzung.

Es hat sich gezeigt, daß die krampflösende und analgetische Wirkung einer erfindungsgemäß verwendbaren pharmazeutischen Zusammensetzung basierend auf Petasitesextrakt mit einem Gehalt an Magnesium im Vergleich zur magnesiumfreien Zusammensetzung wesentlich effektiver ist. So konnte bei der Behandlung von Migräne bei Patienten eine deutlich schneller einsetzende und eine länger andauernde Wirkung der erfindungsgemäßen Petasin-haltigen Zusammensetzung mit Magnesium als mit magnesiumfreien Petasin-haltigen Zusammensetzungen beobachtet werden.

Die pharmazeutische Wirkung von Petasin-haltigen Zusammensetzungen konnte überraschenderweise auch durch Zusatz wenigstens eines pharmazeutisch und/oder physiologisch wirksamen Stoffs, umfassend wenigstens ein Antiphlogistikum bzw. Analgetikum, vorzugsweise Extrakte der Chamomilla recutita, Rhizoma Curcumae longae, Rhizoma Curcumae xanthorrhizae, Curcumae xanthorrhiza, Cortex Salicis, Salicis purpurea, Salicis daphenoides und/oder Tanacetum parthenium; wenigstens ein Spurenelement, vorzugsweise Eisen, Kobalt, Chrom, Iod, Kupfer, Mangan, Zink und/oder Selen; wenigstens ein Sekretolytikum bzw. Sekretomotorikum, vorzugsweise Extrakte der Süßholzwurzel, Thymiankraut und/oder Pfefferminzöl; wenigstens ein Bronchospasmolytikum, vorzugsweise Extrakte von Efeublättern; und/oder Vitamin, vorzugsweise Vitamin A, B, C, D und/oder E, gesteigert werden.

Außerdem wurde gefunden, daß die Resorption der erfindungsgemäß verwendbaren Petasites enthaltenden pharmazeutischen Zusammensetzung im Magen-Darmtrakt erhöht werden kann, wenn man dieser Zusammensetzung wenigstens einen Emulgator, wie Natriumalkylsulfat, Alkylsulfonat, Alkylcarboxylat, Alkylalkoholat, bevorzugt mit einer Alkylkettenlänge von C₁₂-C₁₈;
Polyethylenglykolfettalkoholether, wenigstens einen Ester sowie Ether von Polyethylenglykol mit höheren Fettsäuren bzw. Fettalkoholen und/oder Polyethylenglykolstearat, besonders bevorzugt Cetylstearylalkohol und/oder Glycerin-Polyethylenglycolricinoleat, Lecithin, Sojaöl oder Polysorbate zusetzt.

Eine weitere erfindungsgemäß vorteilhafte Ausführungsform umfaßt die Verwendung einer Petasites enthaltenden Zusammensetzung bei der die Resorption im Magen-Darmtrakt verzögert wird.

Die Verwendung einer solchen Zusammensetzung hat den Vorteil, daß man eine größere Menge an Petasitesextrakt je Dosiseinheit verabreichen kann, die dann über einen verlängerten Zeitraum resorbiert wird. Hierdurch wird es möglich, einen Petasiteswirkstoff-Blutplasmaspiegel der pharmazeutisch aktiven Hauptwirkstoffe des Petasitisextrakts über einen Zeitraum von wenigsten 12 - 48 Stunden, vorzugsweise 24 Stunden zu erreichen, der eine zweimalige, vorzugsweise einmalige Verabreichung der pharmazeutischen Petasites enthaltenen Zusammensetzung am Tag erlaubt.

Geeignete Substanzen hierfür umfassen die die Resorption im Magen-Darmtrakt für die Freisetzung von lipophilen Wirkstoffen allgemein bekannten verzögernden Stoffe. Vorzugsweise ist der die Resorption verzögernde Stoff ausgewählt aus der Gruppe der Paraffine, vorzugsweise ein bei Raumtemperatur festes Paraffin, besonders bevorzugt ein Hartparaffin.

Außerdem kann eine erfindungsgemäß verwendbare oral verabreichbare Petasitesextrakt enthaltene Darreichungsform mit einer Magensaft-resistenten und/oder die Resorption verzögernden Schicht überzogen werden.

Ein besonderer Nachteil des Petasitisextrakts ist dessen unangenehmer, bitterer Geschmack. Infolgedessen ist eine Darreichungsform, die den Wirkstoff bereits im Mund-/Rachenraum teilweise oder vollständig freisetzt, nicht möglich, da der Geschmack des Petasitisextrakts von den Patienten als unangenehm empfunden wird und teilweise sofortige Übelkeit hervorruft.

Es wurde nun gefunden, daß der bittere, äußerst unangenehme Geschmack von Petasites bzw. Petasitesextrakt, durch Zusatz wenigstens eines geschmacksmaskierenden Stoffes wirksam maskiert werden kann, wobei der geschmacksmaskierende Stoff vorzugsweise ätherische Öle, Essenzen, Aromatische Wässer, Ölzucker, Fruchtaromen, wie Erdbeer,

Zitrone, Vanille, und dergleichen, aromatische Drogenextrakte, künstliche Aromastoffe, Zucker, Polyole mit Süßkraft, neutral schmeckende Verdickungsmittel, Cyclodextrine und/oder Mischungen davon, umfaßt.

Durch Zusatz eines geschmacksverbessernden Stoffes lassen sich so Petasitesextrakt enthaltene Tees, Kautabletten, Säfte und dergleichen verabreichen.

Die erfindungsgemäß verwendbare, Petasitesextrakt enthaltende pharmazeutische Zusammensetzung kann in einer flüssigen, festen oder liposomalen Darreichungsform vorliegen. Erfindungsgemäß vorteilhaft ist es, wenn die Darreichungsform ein Spray, ein Aerosol, ein Schaum, ein Inhalat, ein Pulver, eine Tablette, eine Kapsel, eine Weichgelatinekapsel, eine Kautablette, eine Salbe, eine Creme, ein Gel, ein Suppositorium oder eine Injektionslösung ist. Besonders bevorzugt ist es, wenn die erfindungsgemäß verwendbare Petasitesextrakt enthaltende pharmazeutische Zusammensetzung als Tablette, Kapsel oder Kautablette, die den Wirkstoff in Form von Mikrokapseln umfaßt, ausgegebildet ist.

Als mögliche weitere pflanzliche Substanzen für eine erfindungsgemäß verwendbare Petasitescreme sind insbesondere Calendula (tinctura/extraction), Viola (tinctura/extraction) und/oder Vitamine, vorzugsweise Vitamin E geeignet.

Erfindungsgemäß vorteilhaft ist es, wenn die Tagesdosis bevorzugt im Bereich von 5 - 600 mg Petasites Extrakt, besonders bevorzugt im Bereich von 5 - 250 mg Petasites Extrakt liegt.

Die erfindungsgemäß verwendbare Petasitesextrakt enthaltende pharmazeutische Zusammensetzung ist insbesondere zur Behandlung von gastrointestinalen Erkrankungen aller Art, zum Beispiel von endo- und exogenen Schädigungen der Magen- und Darmmukosa, von gastrointestalen Ulzerationen und Gastritiden aller Art, Morbus Crohn und Colitis ulcerosa, sowie von Husten, von Asthma, von Pollinosis, von Ekzemen, von Migräne, von Psoriasis, von Dysmenorrhoe, von Bluthochdruck oder zur Verwendung als Spasmolytikum und Analgetikum geeignet.

Außerdem ist die erfindungsgemäß verwendbare Petasitesextrakt enthaltende pharmazeutische Zusammensetzung zur Herstellung eines Arzneimittels zur Behandlung von Ekzemen und/oder von Psoriasis geeignet.

Die nachfolgenden Beispiele sollen die Erfindung, ohne sie in irgendeiner Weise einzuschränken, näher erläutern:

### Beispiel 1: Unterkritische Propanextraktion von Petasites

500g zerkleinerter Pestwurz-Droge aus Rhizoma Petasitidis mit einer durchschnittlichen Feuchte von 12,8 Gew.-%, einem Petasingehalt von ca. 0,8 % und einem Gehalt an Pyrrolizidin-Alkaloiden von etwa 20-30 ppm wurden im Autoklaven (25 l Volumen) bei einer Temperatur von 24 °C und einem Druck von 13,8 MPa mit ca. 6 kg Propan in einem Schritt extrahiert. Der nach dem Abdampfen des Propans erhaltene trübe Rohextrakt (ca. 17 g) enthielt etwa 10 % Wasser und einen Pyrrolizidin-Alkaloidgehalt von unter 0,1 ppm

### Beispiel 2: Wässrige Aufarbeitung des Rohextrakts

Der Extrakt wird dreimal mit der fünffachen Menge an 0,1 normaler Schwefelsäure ausgeschüttelt, die Säurephase abgetrennt, der Extrakt durch mehrmaliges Waschen mit bidestilliertem Wasser auf einen pH-Wert von 3-7 gebracht und der Extrakt anschließend wieder auf einen Wassergehalt von 1 Gew.-% gebracht. Der so erhaltene Extrakt enthält nach GC-MS-Analyse keine nachweisbaren Pyrrolizidinalkaloide. Dieser Extrakt wird dann 5 mal im Verhältnis von 1 Teil Extrakt und 2 Teilen bidestilliertem Wasser, mit einer Wassertemperatur von wenigstens 45°C ausgeschüttelt. Nach jeder Durchmischung wird die Trennung der Wasserphase von der lipophilen Extraktphase durch Stehenlassen abgewartet und anschließend das überstehende Wasser von oben vorsichtig abgegossen. Im letzten Schritt werden nach dem Dekantieren 20 g Natriumsulfat zugesetzt. Das mit Wasser beladene Natriumsulfat wird abzentrifugiert.
Anschließend wurde ein ELISA zur Bestimmung des Restpyrrolizidinalkaloidgehalts im Petasitesextrakt durchgeführt, hierbei konnte kein Pyrrolizidinalkaloid mehr nachgewiesen werden. Die Nachweisgrenze eines ELISA für Pyrrolizidinalkaloide ist im Vergleich zur GC-MS-Kopplung wenigstens um den Faktor 1000 empfindlicher.

### Beispiel 3: Rezeptur für eine retardierte Weichgelatinekapsel

| Zusammensetzung: | Qualität | Menge |
|---|---|---|
| Wirkstoff | | |
| Extrakt Petasitidis e radice 30:1 | | 25,0 mg |

| Hilfsstoffe | | |
|---|---|---|
| Mittelkettige Triglyceride* | Ph. Eur. 1997 | 245,0 mg |

| Kapselhülle | | |
|---|---|---|
| Glycerol 85% | Ph. Eur. 1997 | 23,52-27,60 mg |
| Trockensubstanz aus Sorbitol-Lösung 70% Nicht kristallin | Ph. Eur. 1997 | 17,12-20,10 mg |
| Gelatine | Ph. Eur. 1997 | 80,89-94,96 mg |
| Eisenoxidrot | E 172 | 0,47-0,55 mg |
| Glycerol | Ph. Eur. 1997 | 1,60-1,88 mg |
| Eisenoxid schwarz | E 172 | 1,13-1,33 mg |

| Überzug | | |
|---|---|---|
| Eudragit RL (nach Fiedler) | | 1,0-15,0 mg |

| | | |
|---|---|---|
| * Weitere Lipoide, in denen sich der Extrakt löst, umfassen: Olivenöl, Maiskeimöl, Sojaöl. Therapeutische Plasmakonzentration 1 bis 100 mg/l. | | |

### Beispiel 4: Rezeptur für eine magensaftresistente Weichgelatinekapsel

| Zusammensetzung: | Qualität | Menge |
|---|---|---|
| Wirkstoff | | |
| Extrakt Petasitidis e radice 30:1 | | 25,0 mg |

| Hilfsstoffe | | |
|---|---|---|
| Mittelkettige Triglyceride* | Ph. Eur. 1997 | 245,0 mg |

| Kapselhülle | | |
|---|---|---|
| Glycerol 85% | Ph. Eur. 1997 | 23,52-27,60 mg |
| Trockensubstanz aus Sorbitol-Lösung 70% Nicht kristallin | Ph. Eur. 1997 | 17,12-20,10 mg |
| Gelatine | Ph. Eur. 1997 | 80,89-94,96 mg |
| Eisenoxid rot | E 172 | 0,47-0,55 mg |
| Glycerol | Ph. Eur. 1997 | 1,60-1,88 mg |
| Eisenoxid schwarz | E 172 | 1,13-1,33 mg |

| Überzug | | |
|---|---|---|
| Celluloseacetatphthalat | | 1,0-15,0 mg |

| | | |
|---|---|---|
| * Weitere Lipoide, in denen sich der Extrakt löst, umfassen: Olivenöl, Maiskeimöl, Sojaöl. Therapeutische Plasmakonzentration 1 bis 100 mg/l. | | |

### Beispiel 5: Injektionslösung

Der Petasitesextrakt wird 1:10 mit hochgereinigtem Sojaöl unter Zusatz von 0,1-2 Gew.-% Eilecithin als Emulgator gemischt und die Mischung zu 0,5 bis 2,0 Gew.-% in Wasser für Injektionszwecke emulgiert.
Therapeutische Plasmakonzentration 1 bis 100 µg/l.

### Beispiel 6: Liposomale Zusammensetzung

Die Liposomen werden aus Glycerophospholipiden, Cholesterol und Stearylamin und wenigstens einem Lipidderivat hergestellt, wobei der Pestwurzextrakt in der Lipidphase der Liposomen gelöst wird.
Therapeutische Plasmakonzentration 1 bis 100 mg/l.

### Beispiel 7: Extrakt Petasites e rad. 30:1 in flüssiger Darreichungsform

| Zusammensetzung 100g enthalten: | Qualität | Menge |
|---|---|---|
| Wirkstoff | | |
| Extrakt Petasitidis e radice 30:1 | | 500 mg |

| Hilfsstoffe | | |
|---|---|---|
| Glycerin-polyethylenglycolricinoleat | USP XXII | 4500 mg |
| Ethanol 43% | HAB 1 | 95,000 g |
| | | 100,000 g |

## Patentansprüche

1. Verwendung einer pharmazeutischen Zusammensetzung basierend auf Petasitesextrakt ohne hepatotoxischer, carcinogener, zytostatischer und/oder mutagener Wirkung, wobei der Petasitesextrakt aus der Pflanze und/oder Pflanzenteilen der Gattung Petasites gewonnen wird zur Herstellung eines Arzneimittels zur Behandlung von Ekzemen und/oder von Psoriasis,
**dadurch gekennzeichnet, dass** der Petasitesextrakt frei von Pyrrolizidinalkaloiden ist, wobei der pharmazeutischen Zusammensetzung gegebenenfalls Magnesium und/oder weitere pharmazeutisch und/oder physiologisch wirksame Stoffe zugesetzt sind.

2. Verwendung einer pharmazeutischen Zusammensetzung nach Anspruch 1,
**dadurch gekennzeichnet, dass** der Extrakt aus Pflanzen und/oder Pflanzenteilen von Petasites hybridus, Petasites albus, Petasites japonicus, Petasites paradoxus, und/oder Petasites spurius gewonnen wird, wobei vorzugsweise die unterirdischen Pflanzenteile zur Herstellung des Extrakts verwendet werden.

3. Verwendung einer pharmazeutischen Zusammensetzung nach Anspruch 1 oder 2,
**dadurch gekennzeichnet, dass** der pharmazeutisch und/oder physiologisch wirksame Stoff ausgewählt ist aus der Gruppe umfassend wenigstens ein Antiphlogistikum bzw. Analgetikum, vorzugsweise Extrakte der Chamomilla recutita, Rhizoma Curcumae longae, Rhizoma Curcumae xanthorrhizae, Curcumae xanthorrhiza, Cortex Salicis, Salicis purpurea, Salicis daphenoides und/oder Tanacetum parthenium; Spurenelemente, vorzugsweise Eisen, Kobalt, Chrom, Iod, Kupfer, Mangan, Zink und/oder Selen; Sekretolytikum bzw. Sekretomotorikum, vorzugsweise Extrakte der Süßholzwurzel, Thymiankraut und/oder Pfefferminzöl; Bronchospasmolytikum, vorzugsweise Extrakte von Efeublättern; Calendula; Viola und/oder Vitamin, vorzugsweise Vitamin A, B, C, D und/oder E.

4. Verwendung einer pharmazeutischen Zusammensetzung nach einem der vorherigen Ansprüche,
**dadurch gekennzeichnet, dass** dem Petasitesextrakt wenigstens ein Stoff zugesetzt ist, der die Resorption im Magen-Darmtrakt erhöht, wobei der die Resorption erhöhende Stoff vorzugsweise einen Emulgator, wie Natriumalkylsulfat, Alkylsulfonat, Alkylcarboxylat, Alkylalkoholat, bevorzugt mit einer Alkylkettenlänge von C₁₂-C₁₈; Polyethylenglykolfettalkoholether, Ester sowie Ether von Polyethylenglykol mit höheren Fettsäuren bzw. Fettalkoholen und/oder Polyethylenglykolstearat; besonders bevorzugt Cetylstearylalkohol und/oder Glycerinpolyethylenglycolricinoleat, umfaßt.

5. Verwendung einer pharmazeutischen Zusammensetzung nach einem der vorherigen Ansprüche,
**dadurch gekennzeichnet, dass** dem Petasitesextrakt wenigstens ein Stoff zugesetzt ist, der die Resorption im Magen-Darmtrakt verzögert, wobei der die Resorption verzögernde Stoff vorzugsweise ausgewählt ist aus der Gruppe der Paraffine, vorzugsweise ein bei Raumtemperatur festes Paraffin ist, besonders bevorzugt ein Hartparaffin ist.

6. Verwendung einer pharmazeutischen Zusammensetzung nach einem der vorherigen Ansprüche,
**dadurch gekennzeichnet, dass** dieser ein geschmacksmaskierender Stoff zugesetzt ist, wobei der geschmacksmaskierende Stoff vorzugsweise ätherische Öle, Essenzen, Aromatische Wässer, Ölzucker, Fruchtaromen, aromatische Drogenextrakte, künstliche Aromastoffe, Zucker, Polyole mit Süßkraft, neutral schmeckende Verdickungsmittel, Cyclodextrine und/oder Mischungen davon, umfasst.

7. Verwendung einer pharmazeutischen Zusammensetzung nach einem der vorherigen Ansprüche,
**dadurch gekennzeichnet, dass** diese in flüssiger, halbfester, fester oder liposomaler Form vorliegt.

8. Verwendung einer pharmazeutischen Zusammensetzung, nach einem der vorherigen Ansprüche,
**dadurch gekennzeichnet, dass** die Darreichungsform ein Spray, ein Aerosol, ein Schaum, ein Inhalat, ein Pulver, eine Tablette, eine Kapsel, eine Weichgelatinekapsel, eine Kautablette, eine Salbe, eine Creme, ein Gel, ein Suppositorium oder eine Injektionslösung ist.

9. Verwendung einer pharmazeutischen Zusammensetzung nach einem der vorherigen Ansprüche,
**dadurch gekennzeichnet, dass** die Tablette, Kapsel und Kautablette aus Mikrokapseln geformt ist.

10. Verwendung einer pharmazeutischen Zusammensetzung nach einem der vorherigen Anspruche,
**dadurch gekennzeichnet, dass** feste oral verabreichbare Darreichungsformen mit einer Magensaft resistenten Schicht, vorzugsweise Celluloseacetatphthalat, versehen sind.

11. Verwendung einer pharmazeutischen Zusammensetzung, nach einem der vorherigen Ansprüche,
**dadurch gekennzeichnet, dass** die Tagesdosis bevorzugt im Bereich von 5-600 mg Petasitesextrakt, besonders bevorzugt im Bereich von 5-250 mg Petasitesextrakt liegt.

## Claims

1. Use of a pharmaceutical composition based on petasites extract without hepatotoxic, carcinogenic, cytostatic and/or mutagenic effect, in which the petasites extract is recovered from plants and/or plant parts of the genus Petasites to produce a drug for treatment of eczemas and/or psoriasis, **characterized in that** the petasites extract is free of pyrrolizidine alkaloids, in which magnesium and/or additional pharmaceutically and/or physiologically active substances are optionally added to the pharmaceutical composition.

2. Use of a pharmaceutical composition according to Claim 1, **characterized in that** the extract is recovered from plants and/or plant parts of Petasites hybridus, Petasites albus, Petasites japonicus, Petasites paradoxus and/or Petasites spurius, in which the underground plant parts are preferably used to produce the extract.

3. Use of a pharmaceutical composition according to Claim 1 or 2, **characterized in that** the pharmaceutically and/or physiologically active substance is selected from the group comprising at least an antiphlogistic or analgesic, preferably extracts of Chamomile recutita, Rhizoma Curcumae longae, Rhizoma Curcumae xanthorrhizae, Curcumae xanthorrhiza, Cortex Salicis, Salicis purpurea, Salicis daphenoides and/or Tanacetum parthenium; trace elements, preferably iron, cobalt, chromium, iodine, copper, manganese, zinc and/or selenium; secretolytic or secretomotor agent, preferably extracts of licorice root, thyme and/or peppermint oil; bronchospasmolytic, preferably extracts of ivy leaves; Calendula; Viola and/or vitamin, preferably vitamin A, B, C, D and/or E.

4. Use of a pharmaceutical composition according to one of the previous claims, **characterized in that** at least one substance is added to the petasites extract that increases resorption in the gastrointestinal tract, in which the substance that increases resorption preferably comprises an emulsifier, like sodium alkyl sulfate, alkyl sulfonate, alkyl carboxylate, alkyl alcoholate, preferably with an alkyl chain length of C₁₂-C₁₈; polyethylene glycol fatty alcohol ethers; esters as well as ethers of polyethylene glycol with higher fatty acids, fatty alcohols and/or polyethylene glycol stearate; especially cetylstearyl alcohol and/or glycerol-polyethylene glycol ricinoleate.

5. Use of a pharmaceutical composition according to one of the previous claims, **characterized in that** at least one substance is added to the petasites extract that delays resorption in the gastrointestinal tract, in which the substance that delays resorption is preferably selected from the group of paraffins, preferably a paraffm solid at room temperature, especially a hard paraffin.

6. Use of a pharmaceutical composition according to one of the previous claims, **characterized in that** a taste-masking substance is added to it, in which the taste-masking substance preferably comprises essential oils, essences, aromatic waters, oil sugars, fruit flavourings, aromatic drug extracts, artificial flavourings, sugars, polyols with sweetening power, neutral-tasting thickeners, cyclodextrins and/or mixtures thereof.

7. Use of a pharmaceutical composition according to one of the previous claims, **characterized in that** it is present in liquid, semisolid, solid or liposomal form.

8. Use of a pharmaceutical composition according to one of the previous claims, **characterized in that** the form of administration is a spray, an aerosol, a foam, an inhalate, a powder, a tablet, a capsule, a soft gelatin capsule, a chewable tablet, a salve, a cream, a gel, a suppository or an injection solution.

9. Use of a pharmaceutical composition according to one of the previous claims, **characterized in that** the tablet, capsule and chewable tablet is formed from microcapsules.

10. Use of a pharmaceutical composition according to one of the previous claims, **characterized in that** the solid orally administerable forms of administration are provided with a layer resistant to gastric juice, preferably cellulose acetate phthalate.

11. Use of a pharmaceutical composition according to one of the previous claims, **characterized in that** the daily dose preferably lies in the range from 5-600 mg petasites extract, especially in the range from 5-250 mg petasites extract.

## Revendications

1. Utilisation d'une composition pharmaceutique à base d'extrait de pétasite en l'absence d'effets hépatotoxiques, carcinogènes, cytostatiques et/ou mutagènes, dans laquelle l'extrait de pétasite est obtenu à partir de la plante et/ou de parties de la plante de l'espèce Petasites pour la préparation d'un médicament destiné au traitement d'eczémas et/ou du psoriasis, **caractérisée en ce que** l'extrait de pétasite est exempt d'alcaloïdes de pyrolizidine, du magnésium et/ou d'autres substances à activité pharmaceutique et/ou physiologique ayant été le cas échéant ajoutées à la composition pharmaceutique.

2. Utilisation d'une composition pharmaceutique selon la revendication 1, **caractérisée en ce qu'**on obtient l'extrait à partir de plantes et/ou de parties de plantes de Petasites hybridus, Petasites albus, Petasites japonicus, Petasites paradoxus et/ou Petasites spurius, les parties souterraines des plantes étant de préférence utilisées pour la préparation de l'extrait.

3. Utilisation d'une composition pharmaceutique selon la revendication 1 ou 2, **caractérisée en ce que** la substance à activité pharmaceutique et/ou physiologique est choisie parmi le groupe comprenant au moins un antiphlogistique, respectivement un analgésique, de préférence des extraits de Chamomilla recutita, Rhizoma Curcumae longae, Rhizoma Curcumae xanthorrhizae, Cortex Salicis, Salicis purpurea, Salicis daphenoides et/ou Tanacetum parthenium ; des oligo-éléments, de préférence le fer, le cobalt, le chrome, l'iode, le cuivre, le manganèse, le zinc et/ou de sélénium ; un agent sécrétolytique, respectivement sécrétomoteur, de préférence des extraits de racine de réglisse, de fanes de thym et/ou d'essence de menthe poivrée ; un bronchospasmolytique, de préférence des extraits de feuille de lierre ; du calendula ; de la violette et/ou des vitamines, de préférence des vitamines A, B, C, D et/ou E.

4. Utilisation d'une composition pharmaceutique selon l'une quelconque des revendications précédentes, **caractérisée en ce qu'**on ajoute à l'extrait de pétasite, au moins une substance qui augmente la résorption dans le système gastro-intestinal, la substance augmentant la résorption comprenant de préférence un émulsifiant tel qu'un alkylsulfate de sodium, un sulfonate d'alkyle, un carboxylate d'alkyle, un alcoolate d'alkyle, de préférence possédant une longueur de chaîne alkyle de 12 à 18 atomes de carbone ; des éthers d'alcools gras de polyéthylèneglycol ; des esters et des éthers de polyéthylèneglycol avec des acides gras respectivement des alcools gras supérieurs et/ou du stéarate de polyéthylèneglycol ; de manière particulièrement préférée de l'alcool cétylstéarylique et/ou du ricinoléate de glycérol-polyéthylèneglycol.

5. Utilisation d'une composition pharmaceutique selon l'une quelconque des revendications précédentes, **caractérisée en ce qu'**on ajoute à l'extrait de pétasite au moins une substance qui ralentit la résorption dans le système gastro-intestinal, la substance ralentissant la résorption étant de préférence choisie parmi le groupe des paraffines, de préférence une paraffine qui est solide à la température ambiante, de manière particulièrement préférée, une paraffine dure.

6. Utilisation d'une composition pharmaceutique selon l'une quelconque des revendications précédentes, **caractérisée en ce qu'**on ajoute à ladite composition, une substance de masquage du goût, la substance masquant le goût comprenant de préférence des huiles éthérées, des essences, des eaux aromatiques, des sucres huileux, des arômes de fruits, des extraits secs aromatiques, des substances aromatisantes synthétiques, des sucres, des polyols à effet sucré, des épaississants à sensation gustative neutre, des cyclodextrines et/ou leurs mélanges.

7. Utilisation d'une composition pharmaceutique selon l'une quelconque des revendications précédentes, **caractérisée en ce que** ladite composition est présente sous une forme liquide, semi-solide, solide ou liposomiale.

8. Utilisation d'une composition pharmaceutique selon l'une quelconque des revendications précédentes, **caractérisée en ce que** la forme d'administration se présente sous la forme d'un spray, d'un aérosol, d'une mousse, d'un produit d'inhalation, d'une poudre, d'un comprimé, d'une capsule, d'une capsule de gélatine molle, d'une tablette à mâcher, d'un onguent, d'une crème, d'un gel, d'un suppositoire ou encore d'une solution pour injection.

9. Utilisation d'une composition pharmaceutique selon l'une quelconque des revendications précédentes, **caractérisée en ce que** le comprimé, la capsule et la tablette à mâcher sont façonnés à partir de microcapsules.

10. Utilisation d'une composition pharmaceutique selon l'une quelconque des revendications précédentes, **caractérisée en ce que** des formes d'administration solides par voie orale sont enrobées d'une couche résistant au suc gastrique, de préférence d'acétophtalate de cellulose.

11. Utilisation d'une composition pharmaceutique selon l'une quelconque des revendications précédentes, **caractérisée en ce que** la dose quotidienne se situe de préférence dans la plage de 5 à 600 mg d'extrait de pétasite, de manière particulièrement préférée dans la plage de 5 à 250 mg d'extrait de pétasite.
